# EUROPEAN PATENT APPLICATION

(11) **EP 4 287 142 A1**
(43) Date of publication of application: **06.12.2023**
(21) Application number: 22290038.3
(22) Date of filing: 03.06.2022
(51) Int. Cl.: G06V 10/82, G06K 9/00, G06V 10/44, G06V 20/70, G06V 20/90, G16H 50/20

(54) **DEEP LEARNING MODELS OF RADIOMICS FEATURES EXTRACTION**

(71) Applicant: Sophia Genetics SA, 1025 Saint-Sulpice (CH)
(72) Inventor: Colin, Thierry, 1025 St-Sulpice (CH); Huc, Antoine, 1025 St-Sulpice (CH); Gallinato, Olivier, 1025 St-Sulpice (CH); Siffre, Jason, 1025 St-Sulpice (CH); Gidel, Floriane, 1025 St-Sulpice (CH)
(74) Representative: Wenger, Joel-Théophile

(57) **Abstract**

A multi-purpose automated radiomics workflow is proposed which facilitates the extraction of a bank of normalized radiomics features for a diversity of imaging modalities and patient pathologies. It is versatile enough to provide a robust extraction of the normalized features without requiring manual corrections even when the patient pathology is characterized by multisite lesions and thus multiple segmented sites, such as for instance multiple tumors or metastasis sites in late cancer stages.

## Description

### Field

The present invention relates to the field of medical imaging data processing. In particular, the present invention relates to the deep learning predictive models of a radiomics signature for patients and to the computer-implemented methods of extracting and refining radiomics features from medical images.

### Background

Emerging patient care data processing platforms enable the generation of high-throughput quantitative imaging features for a diversity of medical applications in clinical practice. This features can be used to support a number of clinical applications from diagnosis to the choice of treatment and prognosis, in particular the increasing medical demand for personalized medicine in oncology. In this context, radiomics has emerged as a discipline for helping clinicians move beyond limited medical imaging gold standard response criteria. Streamlined, end-to-end, automated radiomics workflows aim at segmenting medical images, extracting standardized radiomics features and correlating them with outcomes.

In this context radiomics research is booming - in 2020, more than 1,500 peer-reviewed radiomics papers were published (Pinto dos Santos et al. (2021), European Radiology, 31, pp1-4). In oncology, the use of a radiomic signature based on the extraction of radiomics features from patient medical imaging have recently been shown to facilitate the prediction of efficacy of immunotherapy in lung cancer (Sun et al (2018), The Lancet Oncology, Vol. 19 (9):1180-1191*;* Tunali et al. (2019), J Thoracic Oncology Vol. 14 (11) Sup 1 S1129*;* Mu et al. (2020), Eur J Nucl Med Mol Imaging 47(5):1168-1182).

Given the importance of radiomics for a diversity of clinical applications, initiatives have recently emerged to standardize the radiomic analysis and features extraction, such as the Imaging Biomarker Standardization Initiative (IBSI - https://theibsi.github.io/ and Zwanenburg et al. (2016) - eprint arXiv:1612.07003). These standards rely upon the computer-implemented extraction of several hundreds of radiomics features such as features related to:
- morphological features characterizing the shapes of objects of interest, for instance area, volume, compactness, or sphericity of tumor objects in the images;
- first order features such as luminance and color intensity histograms;
- second-order statistics features such as textural features, for instance GLCM (Grey Level Co-occurrence Matrix) or Haralick features;
as well as higher order statistical features extracted by various signal processing methods from the image data, for instance digital signal filters and frequency domain transforms to better characterize certain image patterns.

The feature extraction image analysis and image processing algorithms can be implemented in medical imaging software platforms, taking as input the medical imaging data to produce a bank of radiomic features extracted from the medical images. A radiomics workflow to extract a bank of features from segmentation in imaging data in combination with machine learning classifiers such as decision trees and support vector machines (SVM) was described in WO2007/079207. Fornacon-Wood et al. European Radiology (2020) 30:6241-6250, reported 14 radiomics software platforms cited in literature, including the widely used pyradiomics open-source python package (https://pyradiomics.readthedocs.io/en/latest/index.html and van Griethuysen et al. (2017) Cancer Research, 77(21), e104-e107) and other open-source packages. However, there is no golden standard way of ensuring reliability and harmonization of the features calculations due to the multiple modes of parametrizing these algorithms. More recently, the use for more specific applications such as oncology of advanced automated AI methods such as those based on deep learning predictive models (DL) was investigated. It was shown that integration of this methods as part of end-to-end radiomics workflow allows to robustly select most relevant radiomics features. Papadimitroulas et al., Physica Medica 83 (2021), 108-121 provides a review of radiomics extraction and neuron networks DL models for image analysis in oncological radiomics. These models enable two major improvements over the prior art:
1) to refine the extraction of multiple radiomics features from input medical images, in particular by using DL models for image segmentation (instead of a manual or semi-automated segmentation);
2) to select the most relevant features ("robust radiomics features") suitable for a given purpose, such as the prediction of a specific treatment response.
In clinical practice, the medical team may wish to evaluate different treatment options, for instance immunotherapy treatments and other treatments, based on the patient omics, including but not limited to the patient radiomics features. Prediction models are developed specifically for a given application (e.g. a given treatment), and recent work use different subsets of the radiomics features extracted from the patient imaging data: for instance, the radiomics features developed *Sun et al (2018)* are not the same as the radiomics features developed by *Mu et al. (2020).*

In order to optimize the cost of the imaging data processing and storage infrastructure for medical centers along the patient care journey, there is also a need for radiomics extraction systems and workflows which can produce and store.a. bank of radiomics features to be later retrieved and used by an application specific solution. In some applications, for instance in longitudinal follow-ups of a patient over multiple evaluation time points along his/her care journey, this avoids to repeat the whole radiomics features extraction process from the patient imaging data in the PACS system when the medical team is looking into new predictions from the integration of the former radiomics data in the longitudinal analysis.

There is also a need to prepare radiomics features for integration into a fully automated radiomics system and workflow as part of a personalized medicine omics platform which facilitates the diagnosis, the prognosis, the prediction of the treatment response and/or the prediction of the treatment efficacy over time for a specific patient, based on his or her medical imaging data characteristics, while optimizing the resource allocation spend of healthcare systems.

Another emerging problem is the increasing demand for longitudinal evaluations of the patient pathology evolution at different times along the patient healthcare journey. There is therefore a further need for more radiomics features extraction and storage which allow later retrieval and differential comparisons of longitudinal imaging for a given patient, even in the most challenging advanced cancer stages with new multisite lesions to characterize in the later stage imaging data.

### Summary

It is proposed a computer-implemented method is described for producing a bank of radiomics features from the radiological imaging data of a patient, comprising:
a) receiving radiological imaging data of the patient from a first database of medical data;
b) localizing, from the radiological imaging data, at least one anatomical area of interest to produce an anatomical area label;
c) identifying, from the radiological imaging, a set of imaging signal properties;
d) for each anatomical area label, selecting a segmentation method for segmenting the radiological imaging data according to the imaging signal properties;
e) segmenting, with the selected segmentation method, the radiological imaging data to produce one or more connected components collectively forming an object of interest to characterize a pathology of the patient;
f) extracting a bank of normalized radiomics features from the connected components;
g) storing the bank of normalized radiomics features in a second database of digital health features.

In a possible embodiment, localizing, from the radiological imaging data, at least one anatomical area of interest comprises acquiring a localization model trained to extract an anatomical area label from radiological imaging data and applying the localization model to the radiological imaging data to produce an anatomical area label.

In a possible embodiment, identifying, from the radiological imaging data, a set of imaging signal properties comprises acquiring a signal identification model trained to extract a set of imaging signal properties from radiological imaging data and applying the signal identification model to the radiological imaging data to produce a set of imaging signal properties.

The segmentation method may be a manual method, a semi-automated method, or an automated method. In a possible embodiment, the segmentation method is an automated method, comprising acquiring a segmentation model trained to extract from radiological imaging data one or more connected component and applying the segmentation model to the radiological imaging data to produce one or more connected components.

In a possible embodiment, the segmentation method produces a plurality of connected components and extracting a bank of normalized radiomics features from the connected components comprises extracting a summarized feature from the totality of the connected components to produce a normalized feature value; and integrating the normalized feature value into the bank of normalized radiomics features.

In a possible embodiment, the segmentation method produces a plurality of connected components and extracting a bank of normalized radiomics features from the connected components comprises extracting a component-specific feature from each of the connected components; averaging the component-specific features to produce a normalized feature value; and integrating the normalized feature value into the bank of normalized radiomics features. In a possible embodiment, the segmentation method produces a plurality of connected components and extracting a bank of normalized radiomics features from the connected components comprises measuring a size of each connected component to identify the largest connected component; extracting a component-specific feature for the largest component to produce a normalized feature value; and integrating the normalized feature value into the bank of normalized radiomics features.

In a possible embodiment, the normalized feature is a morphological feature, the segmentation method produces a plurality of connected components, and extracting a bank of normalized radiomics features from the connected components comprises extracting a summarized feature from the totality of the connected components to produce a first normalized feature value; extracting a component-specific feature from each of the connected components; averaging the component-specific features to produce a second normalized feature value; measuring a size of each connected component to identify the largest connected component, and selecting the component-specific feature of the largest component to produce a third normalized feature value; integrating the first, second and third normalized values into the bank of normalized features.

In a possible embodiment, the patient pathology is a cancer and each connected component corresponds to a different tumor or metastasis site. In a possible embodiment, the pathology is a non-small cell lung cancer (NSCLC) at stage IV, the anatomical area of interest is lung, and at least one of the connected components corresponds to a lung tumor or metastasis. In a possible embodiment, the pathology is a non-small cell lung cancer (NSCLC) at stage IV, the anatomical area of interest is liver, and at least one of the connected components corresponds to a liver metastasis.

In a possible embodiment, the patient pathology is an infectious disease, for instance SARS-COV2, the anatomical area of interest is lung and at least one of the connected components corresponds to a different ground glass opacity site.

### Description of the figures

The present description will be better understood with the help of the attached figures, namely:
**Figure 1** shows a radiomics processing system.
**Figure 2** shows a radiomics processing workflow.
**Figure 3a****)** shows an example of a segmented site from lung cancer imaging data in 2D and **Figure 3b****)** shows it in 3D.
**Figure 4** illustrates two possible radiomics feature extraction workflows to process multiple segmented sites corresponding to different lesion locations of tumors and metastases as may be observed in some late stage cancers, respectively for a) a non-morphological feature and b) a morphological feature.

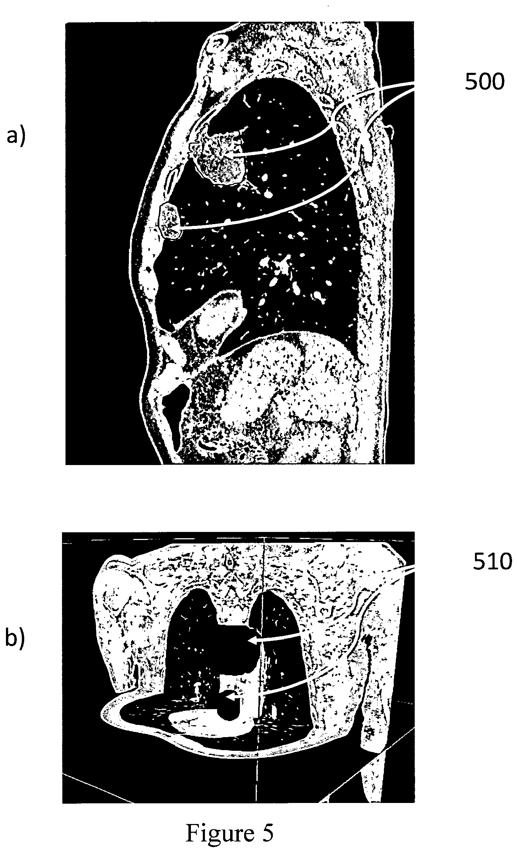

**Figure 5a****)** shows an example of a segmented multi-lesion site from lung cancer imaging data in 2D and **Figure 5b****)** shows it in 3D.

### Detailed description

The term "**medical image data**" or "**radiological data**" or "**imaging data**" or "**radiolological imaging data**" refers to the digital images data, comprising one or more image files and their metadata, as can be collected and archived for a patient at any time point in its health care path journey. These images may be acquired from the patient examination in one or more medical centers in charge with one or more imaging modality such as CT, PET, MRI, SPECT, ultrasound, X-rays, and others. The images may be in 2D or 3D format. These images may be securely collected, stored, archived and transmitted to the radiomics processing system of the invention in accordance with the PACS (Picture Archiving and Communication System) and DICOM (Digital Imaging and Communications in Medicine) digital medical imaging technology standards that are widely deployed in healthcare organizations worldwide.

The term **"radiomics"** as an abbreviation of **"radiology omics"** refers to the high-throughput digital extraction of mineable, quantitative data from radiological imaging data.

The term "**feature**"**,** "**indicator**"**,** "**descriptor**"**,** "**imaging feature**" or "**radiomics feature**" refers to an imaging biomarker which can be extracted from imaging data as quantifiable summarization of the image, for instance a statistical value. The radiomics features or radiomics descriptors refer to a set of values computed from the segmentation of an image area (2D Region of Interest ROI or 3D Volume of Interest VOI), using the intensity values of the 2D pixels or 3D voxels included in the segmented ROI or VOI site. In a radiomics workflow, multiple features, each representative of a different characteristic of the ROI or VOI segmented site in the image, may be individually extracted with a computer-implemented method and combined to produce a bank of features or a radiomics signature. This bank of features may include, but is not limited to heterogeneity, morphological (0-order), intensity (1st order), texture (2^{nd} order) or higher order features. Example of banks of features commonly used in radiomics include any of the 169 well established features of the IBSI1 standard, any of the 1500 features of the Pyradiomics open source software package, and/or any of the LIFEx (www.lifexsoft.org), CERR, or IBEX public software tools. Preferably, radiomics features are represented as scalar values shifted and rescaled so that they fit into the range between 0 and 1 ("**normalized features**")

The term "**localization**" refers to the identification of a target anatomy element, for instance an anatomical area , an organ or part of an organ to be segmented in the medical images. Preferably, localization refers to the identification of an organ or part of an organ, with a digital label suitable for the annotation of the medical images metadata in computer-implemented processing.

The term "**segmentation**" refers to the delineation of an object of interest or a site of interest - Region of Interest (ROI) in 2D, Volume of Interest (VOI) in 3D - to extract object shapes from a localized anatomy area. Segmentation may be achieved either manually by an operator; or semi-automatically by an image processing computer-implemented software under closed supervision, configuration and parametrization by an operator (for instance, using the ImageJ software suite tools such as the region based methods, the graph-based methods, the shape-based methods, the morphological methods, the clustering methods, the thresholding methods and others from *https:*//*imagej.net*); or automatically using a machine learning segmentation model, such as for instance a deep-learning model of image segmentation. In the case of a tumor, segmentation enables to extract all of the various areas inside and around the tumor (also called habitats), which vary with the tumor characteristics such as blood flow, cell density, etc. In the case of a metastatic tumor, or more generally in the case of a multisite lesion to characterize in relation with some pathologies, segmentation enables to extract each lesion site to produce a set of **connected components** collectively forming the object of interest.

The term "**feature extraction**" refers to the signal processing analysis and/or calculation of a quantifiable value, such as a normalized feature between 0 and 1, from a digital signal input, such as an image or one or more connected components from a segmented object of interest in an image.

### Radiological imaging data

Radiological imaging data are images collected and include, but are not limited to CT, PET, PET/CT MRI, SPECT.

Radiological imaging data for the patient may include, but are not limited to the patient's imaging at pre-baseline, at baseline, at first evaluation, or at any further evaluation time in a longitudinal follow-up.

Radiological imaging data for a cancer patient may include, but are not limited to the patient's: millimetric injected CT cancer site scan at portal time, PET/CT, CT, MRI, SPECT.

In one embodiment, the radiological imaging data for a patient with a lung cancer, in particular with stage IV NSCLC, may comprise at least one or consist of the following: millimetric injected CT thoracic, abdomen and pelvis scans at portal time, PET/CT, brain CT, brain MRI; chest CT scan; CT-TAP scan; brain CT scan or PET/ CT.

### Radiomics feature extraction system

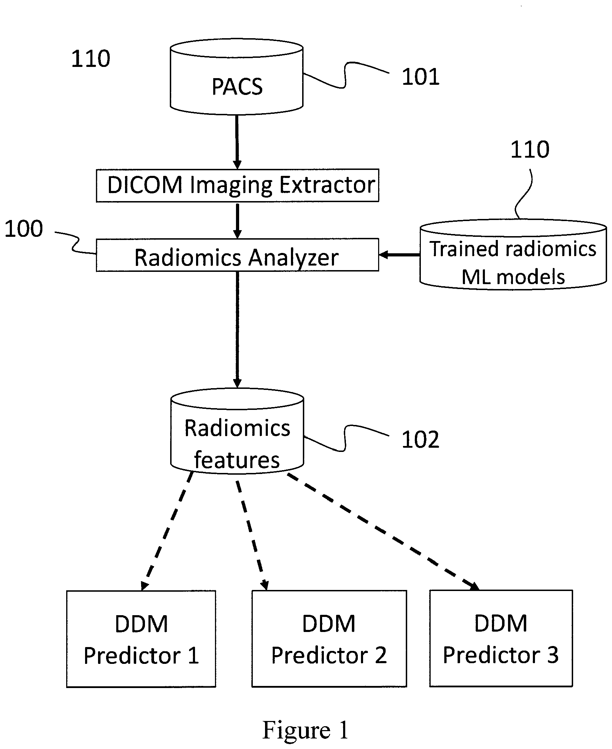

Figure 1 illustrates a radiomics feature extraction system as may be integrated within an end-to-end patient care omics or multi-omics platform. In a medical center image archival system, possibly as part of an electronic healthcare records (EHR) system, radiological imaging data may be stored in a first database 101 of medical data, for instance in a database of medical imaging data in accordance with the PACS/DICOM standards.

For a given patient identifier (possibly an anonymized identifier), archived radiological imaging data may be extracted from the medical imaging data with a DICOM compliant imaging extractor. The radiological imaging data comprises one or more radiological images and possibly some associated metadata information. The metadata usually comprises at least information on the date of the imaging examination and a patient identifier, preferably anonymized. It may also comprise any first level image property parameters from the imaging examination, such as the imaging modality (MRI, CT...), some first level imaging parameters specific to the modality, possibly the imaging equipment manufacturer and version information, and other metadata information directly available from the imaging examination. The imaging extractor transmits the retrieved radiological imaging data to a radiomics analyzer 100 which extracts radiomics features and stores them as a bank of features for the patient into a second database of digital health features. The bank of radiomics features of a patient may be used by different applications at different times, depending on the patient pathology and status. In particular, the bank of radiomics features may be accessed and used at a later time by a data-driven medicine DDM platform (not represented) to predict, with one or more prediction models, a diagnosis or a prognosis for the patient. DDM Predictors for the patient may include, but are not limited to: a predictor of a diagnosis of a pathology; a predictor of a stage of a pathology; a predictor of a prognosis; a predictor of a treatment response; a predictor of a treatment efficacy. Examples of pathologies may include, but are not limited to: a cancer; a neurological disease; a cardiac disease; a musculoskeletal disease; an infectious disease. The DDM Predictors may employ the radiomics features extracted from the patient's imaging at pre-baseline, at baseline, at first evaluation, or at any further evaluation time in a longitudinal follow-up.

In a preferred embodiment, the Radiomics Analyzer 100 is at least partly automated by employing one or more trained radiomics models 110 to facilitate:
- the pre-processing of the medical imaging data, such as annotation, using partial or fully automated localization and/or signal identification;
- and/or the analysis of the medical imaging data, such as segmentation of lesion sites of interest.

Examples of AI techniques trained models 110 to facilitate a specific step of the pre-processing and/or the analysis of the medical imaging data are described for instance in Du et al. (2020) Proc. Machine Learning Research 121:1 74-192 for extraction of localization information from unlabeled DICOM datasets, as well as in the review of Papadimitroulas et al., Physica Medica 83 (2021), 108-121*.*

The Radiomics Analyzer 100 may be a computer-system or part of a computer-system including a central processing unit (CPU, "processor" or "computer processor" herein), memory such as RAM and storage units such as a hard disk, and communication interfaces to communicate with other computer systems through a communication network, for instance the internet or a local network. Examples of radiomics data analyzer computing systems, environments, and/or configurations include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, graphical processing units (GPU), and the like. In some embodiments, the computer system may comprise one or more computer servers, which are operational with numerous other general purpose or special purpose computing systems and may enable distributed computing, such as cloud computing, for instance in a medical data farm. In some embodiments, the radiomics analyzer 100 may be integrated into a massively parallel system. In some embodiments, the radiomics data analyzer 100 may be directly integrated into a next generation sequencing system.

The Radiomics Analyzer 100 computer system may be adapted in the general context of computer system-executable instructions, such as program modules, being executed by a computer system. Generally, program modules may include routines, programs, objects, components, logic, data structures, and so on that perform particular tasks or implement particular abstract data types. As is well known to those skilled in the art of computer programming, program modules may use native operating system and/or file system functions, standalone applications; browser or application plugins, applets, etc.; commercial or open source libraries and/or library tools as may be programmed in Python, Biopython, C/C++, or other programming languages; custom scripts, such as Perl or Bioperl scripts.

Instructions may be executed in distributed cloud computing environments where tasks are performed by remote processing devices that are linked through a communications network. In a distributed cloud-computing environment, program modules may be located in both local and remote computer system storage media including memory storage devices.

It is thus understood that methods described herein are computer-implemented methods.

### Radiomics features extraction workflow

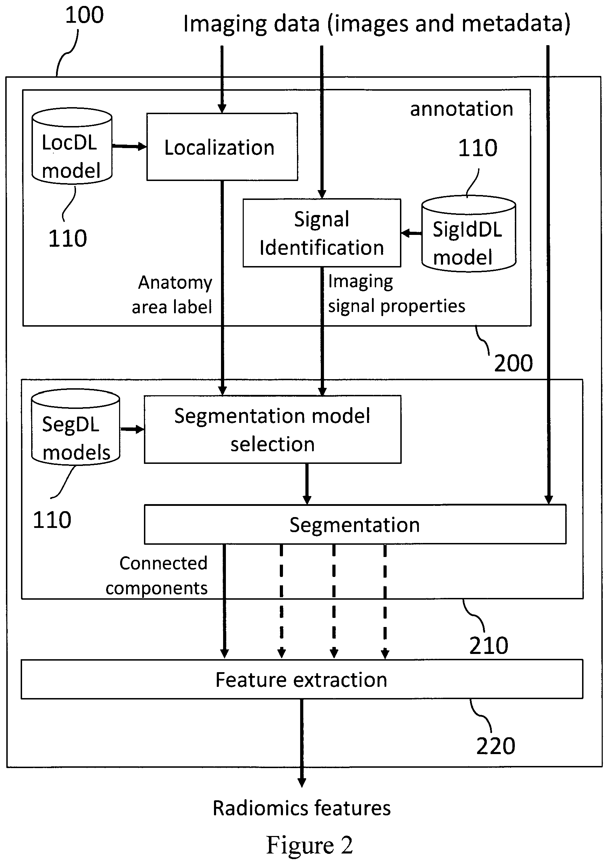

Figure 2 shows a possible radiomics feature extraction workflow for extracting radiomics features of a patient according to some embodiments of a semi-automated or fully automated radiomics analyzer 100 as described herein.

In one embodiment, is provided a computer program comprising instructions which, when the program is executed by a computer, cause the computer to carry out the method of extracting radiomics features of a patient as described herein.

In one embodiment, is provided a computer-readable medium comprising instructions which, when executed by a computer, cause the computer to carry out the method of extracting radiomics features of a patient as described herein.

In one embodiment, the radiomics analyzer 100 receives, from a first database of medical data, radiological imaging data comprising medical images and metadata from a patient examination. The patient radiological imaging data is first processed by an annotation module 200 adapted to extract an anatomy area label and a set of imaging signal properties from the patient radiological imaging data. The patient radiological data images are then segmented by a segmentation module 210 to extract one or more connected components corresponding to an object of interest in the images. Finally, the radiomics analyzer extracts 220 a set of radiomics features from the connected components.

The annotation module may employ a trained machine learning model 110, preferably a deep learning model (LocDL), to automatically identify the anatomy area label. As will be apparent to those skilled in the art of radiomics machine learning, the machine learning model may be trained using a predetermined set of images for which manually annotated anatomy area labels are available (for instance "lung", "liver", "kidney", "brain"...). The anatomy area label may also further comprise information on the patient orientation and view and/or the scan target and coverage.

The annotation module may employ a trained machine learning model 110, preferably a deep learning model (SigIdDL), to automatically identify the set of imaging properties. As will be apparent to those skilled in the art of radiomics machine learning, the machine learning model may be trained using a predetermined set of images for which manually annotated imaging signal properties are available. In a possible embodiment, the imaging signal properties may comprise:
- the 1^{st} level imaging signal parameters such as the imaging modalities and sequences (CT, MRI, PET...), as the latter are not always properly documented in a standard metadata format in DICOM systems;
- and/or the 2^{nd} level imaging signal parameters such as for instance the use of a CT hard or soft filter, the presence of a contrast agent for MRI or CT imaging, the time of acquisition (arterial, portal, washout...), the voxel sizes, the slice spacing, the identification of an MRI T1 or T2 weighting...

In a radiomics analyzer workflow, the segmentation module 210 may employ various segmentation methods to segment the patient radiological images. In a possible embodiment, the segmentation module may select a trained machine learning model 110, preferably a deep learning model (SegDL), to automatically segment the patient radiological images. As will be apparent to those skilled in the art of radiomics segmentation, there is no universal segmentation model adapted to multiple anatomy areas and to the diversity of imaging signal properties as may be identified from an automated annotation workflow. Therefore, the segmentation module 210 may rather comprise a library of segmentation models 110, each corresponding to a specific anatomy area in combination with a specific set of imaging signal properties. As will be apparent to those skilled in the art of radiomics machine learning, each machine learning segmentation model may then be trained using a predetermined set of images covering the same anatomy area and captured with the same set of imaging properties. The segmentation module 210 may then select the segmentation model which matches the localized anatomy area label and the set of imaging properties as extracted from the annotation module 200, and segment, with the selected segmentation model, the patient radiological images to produce one or more connected components.

In a possible embodiment (not represented), the segmentation module 210 may also identify that there is no suitable trained machine learning model available in the library of machine learning models 110 matching the anatomy area and the set of imaging properties. The radiomics analyzer may then select a manual or a semi-automated segmentation method and ask for manual or semi-manual supervision from a user, using the radiomics analyzer user interface.

### Features extraction

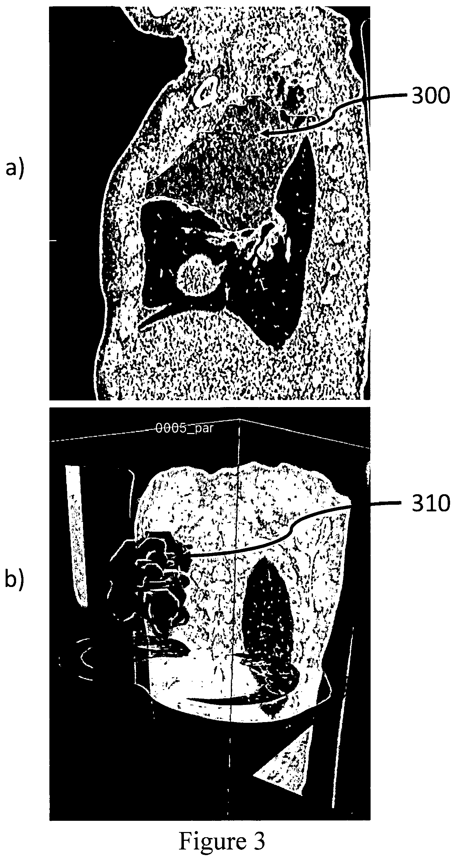

Figure 3 shows an example of a 2D lung cancer image and the corresponding 3D reconstruction image comprising a single tumor lesion 300, 310. In this example, the segmented object of interest is a tumor and may be represented as a ROI (2D) 300 and reconstructed as a VOI (3D) 310 for display to a user using a graphical user interface. In the automated radiomics analyzer 100 workflow, the object of interest may be represented as a set of one or more connected components, each corresponding to a different lesion site. As will be apparent to those skilled in the art, when a single connected component is extracted from segmentation, prior art methods of feature extraction, for instance a conventional IBSI compliant workflow, may be employed to calculate various radiomics features and produce a bank of normalized radiomics features extracted from the patient radiological imaging data. However, when multiple connected components are present out of the segmentation, the conventional feature extraction workflows process them globally to produce radiomics features for the totality of them. In a possible embodiment, the radiomics analyzer 100 extracts 220 one or more normalized features from the connected components to produce a bank of normalized features. The radiomics analyzer 100 may then store the extracted bank of normalized features in a database 102 of digital health features for the patient.

In one embodiment, the radiological imaging data include data on multiple lesion sites. Each normalized feature may be computed for the patient pathology by calculating the feature globally as a summarized feature from the multiple connected components. Alternately, each normalized feature may also be computed from the individual calculation of the features for each segmented site of the patient pathology lesions separately.

In general, it is useful to use separate morphological descriptors when treating a multi-site annotation as the morphological features have been developed in research for the characterization of the morphology of per-site segmented object shapes (major, minor and least axes for instance). In one embodiment, morphological features are extracted by different methods to better describe the morphological state of a group of lesions (multi-site segmentation), such as selected from, but not limited to:
1. Total feature: The feature is computed for the whole multi-site lesion, by aggregating the connected components into a single component prior to feature calculation.
2. Mean feature: The feature is computed for each connected component individually, then, it is averaged.
3. Max feature: The size of each connected component is measured (for instance, as the connected component area, or its largest diameter, or its volume, or its intensity in a PET scan), and only the feature computed from the largest (volume-wise) site is used.

In one embodiment, all the radiomics features are computed for each of the three above-mentioned categories (total feature, mean feature and max feature).

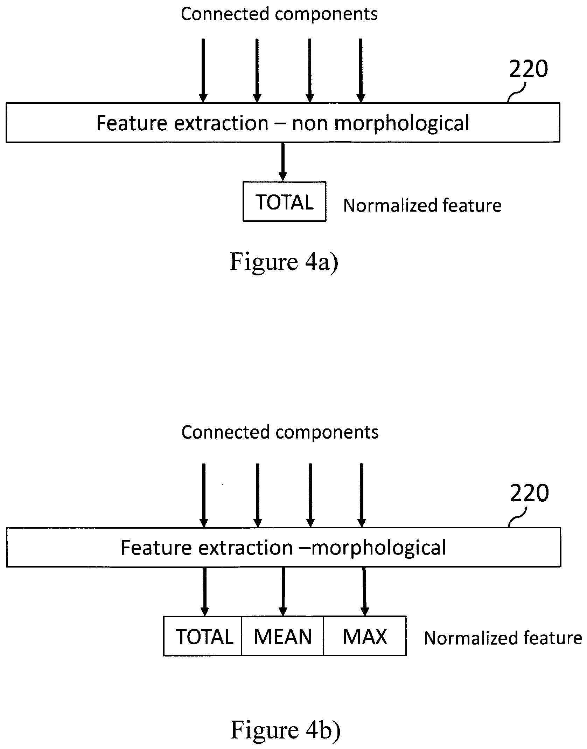

In another embodiment, as illustrated by Figure 4, for non-morphological features (Figure 4a) only the total feature is calculated, while for morphological features (Figure 4b) all the radiomics features are computed for each of the three above-mentioned categories (total feature, mean feature and max feature).

The bank of normalized features produced by the radiomics feature analyzer 100 may then comprise aggregated total, max and mean feature values for one or more of the normalized features records.

In a possible embodiment, extracting a bank of normalized radiomics features from the connected components comprises extracting a summarized feature from the totality of the connected components to produce a normalized feature value; and integrating the normalized feature value into the bank of normalized radiomics features.

In a possible embodiment, extracting a bank of normalized radiomics features from the connected components comprises extracting a component-specific feature from each of the connected components; averaging the component-specific features to produce a normalized feature value; and integrating the normalized feature value into the bank of normalized radiomics features.

In a possible embodiment, extracting a bank of normalized radiomics features from the connected components comprises measuring a size of each connected component to identify the largest connected component; extracting a component-specific feature for the largest component to produce a normalized feature value; and integrating the normalized feature value into the bank of normalized radiomics features.

In a possible embodiment, extracting a bank of normalized radiomics features from the connected components comprises:
- extracting a summarized feature from the totality of the connected components to produce a first normalized feature value;
- extracting a component-specific feature from each of the connected components and averaging the component-specific features to produce a second normalized feature value;
- measuring a size of each connected component to identify the largest connected component, and selecting the component-specific feature of the largest component to produce a third normalized feature value;
- integrating the first, second and third normalized values into the bank of normalized features.

In a possible embodiment, extracting a bank of normalized radiomics features from the connected components comprises:
- For non-morphological features, extracting a summarized feature from the totality of the connected components to produce a normalized feature value;
- For morphological features:
   - extracting a summarized feature from the totality of the connected components to produce a first normalized feature value;
   - extracting a component-specific feature from each of the connected components;
   - averaging the component-specific features to produce a second normalized feature value;
   - measuring a size of each connected component to identify the largest connected component, and selecting the component-specific feature of the largest component to produce a third normalized feature value;
   - integrating the first, second and third normalized values into the bank of normalized features.

### Exemplary applications

In a particular embodiment, the patient pathology is a cancer and the radiological imaging data include data on multiple cancer lesion sites. In particular, the patient's lesion of a diseased tissue may have uneven morphology or spread to other sites, and it is referred herein as a multi-site lesion.

For example, the patient's cancer lesion may have uneven morphology or spread to form metastatic cancer sites and it is referred herein as a multi-site lesion. Examples of multi-lesion cancers are the stage IV of non-small cell lung cancer with liver metastases. Each connected component corresponds to a different tumor or metastasis site. The patient radiological imaging data may comprise two exams, one for the lung, one for the liver. From the lung images, the radiomics analyzer labels the localized anatomical area as "lung", and extracts the radiomics features from at least one of the connected component corresponding to a lung tumor or metastasis. From the liver images, the radiomics analyzer labels the localized anatomical area as "liver", and extracts the radiomics features from at least one of the connected component corresponding to a liver metastasis.

In another particular embodiment, the patient pathology is an infectious disease and the radiological imaging data include data on multiple infectious lesion sites. In particular, the patient's lesion of a diseased tissue may have uneven morphology or spread to other sites, and it is referred herein as a multi-site lesion. For example, the patient's SARS-COV2 lung lesions may have uneven morphology through multiple ground glass opacity site sites and it is referred herein as a multi-site lesion. The patient radiological imaging data may comprise a lung imaging exam. From the lung images, the radiomics analyzer labels the localized anatomical area as "lung", and extracts the radiomics features from at least one of the connected component corresponding to a ground glass opacity site.

## Claims

1. A computer-implemented method for producing a bank of radiomics features from the radiological imaging data of a patient comprising:
h) receiving radiological imaging data of the patient from a first database of medical data;
i) localizing, from the radiological imaging data, at least one anatomical area of interest to produce an anatomical area label;
j) identifying, from the radiological imaging data, a set of imaging signal properties;
k) for each anatomical area label, selecting a segmentation method for segmenting the radiological imaging data according to the imaging signal properties;
l) segmenting, with the selected segmentation method, the radiological imaging data to produce one or more connected components collectively forming an object of interest to characterize a pathology of the patient;
m) extracting a bank of normalized radiomics features from the connected components;
n) storing the bank of normalized radiomics features in a second database of digital health features.

2. The computer-implemented method of claim 1, wherein localizing, from the radiological imaging data, at least one anatomical area of interest comprises:
b1) acquiring a localization model trained to extract an anatomical area label from radiological imaging data;
b2) applying the localization model to the radiological imaging data to produce an anatomical area label.

3. The computer-implemented method of claims 1 or 2, wherein identifying, from the radiological imaging data, a set of imaging signal properties comprises:
c1) acquiring a signal identification model trained to extract a set of imaging signal properties from radiological imaging data;
c2) applying the signal identification model to the radiological imaging data to produce a set of imaging signal properties.

4. The method of claims 1, 2 or 3, wherein the segmentation method is a manual method, a semi-automated method, or an automated method.

5. The method of claim 4, wherein the segmentation method is an automated method, comprising
e1) acquiring a segmentation model trained to extract from radiological imaging data one or more connected components;
e2) applying the segmentation model to the radiological imaging data to produce one or more connected components.

6. The method of any of the claims 1 to 5, wherein the segmentation method produces a plurality of connected components and wherein extracting a bank of normalized radiomics features from the connected components comprises:
- extracting a summarized feature from the totality of the connected components to produce a normalized feature value;
- integrating the normalized feature value into the bank of normalized radiomics features.

7. The method of any of the claims 1 to 6, wherein the segmentation method produces a plurality of connected components and wherein extracting a bank of normalized radiomics features from the connected components comprises:
- extracting a component-specific feature from each of the connected components;
- averaging the component-specific features to produce a normalized feature value;
- integrating the normalized feature value into the bank of normalized radiomics features.

8. The method of any of the claims 1 to 7, wherein the segmentation method produces a plurality of connected components and wherein extracting a bank of normalized radiomics features from the connected components comprises:
- measuring a size of each connected component to identify the largest connected component;
- extracting a component-specific feature for the largest component to produce a normalized feature value;
- integrating the normalized feature value into the bank of normalized radiomics features.

9. The method of any of the claims 1 to 5, wherein the normalized feature is a morphological feature, wherein the segmentation method produces a plurality of connected components and wherein extracting a bank of normalized radiomics features from the connected components comprises:
- extracting a summarized feature from the totality of the connected components to produce a first normalized feature value;
- extracting a component-specific feature from each of the connected components, and averaging the component-specific features to produce a second normalized feature value;
- measuring a size of each connected component to identify the largest connected component, and selecting the component-specific feature of the largest component to produce a third normalized feature value;
- integrating the first, second and third normalized values into the bank of normalized features.

10. The method of any of the preceding claims, wherein the patient pathology is a cancer and each connected component corresponds to a different tumor or metastasis site.

11. The method of claim 10, wherein the pathology is a non-small cell lung cancer (NSCLC) at stage IV, the anatomical area of interest is lung, and at least one of the connected component corresponds to a lung tumor or metastasis.

12. The method of claim 10, wherein the pathology is a non-small cell lung cancer (NSCLC) at stage IV, the anatomical area of interest is liver, and at least one of the connected components corresponds to a liver metastasis.

13. The method of any of the claims 1 to 9, wherein the patient pathology is an infectious disease, the anatomical area of interest is lung and at least one of the connected components corresponds to a different ground glass opacity site.

14. The method of claim 13, wherein the patient pathology is SARS-Cov2.
